# EUROPÄISCHE PATENTSCHRIFT

(11) **EP 1 179 536 B1**
(45) Veröffentlichungstag und Bekanntmachung des Hinweises auf die Patenterteilung: **16.01.2008**
(21) Anmeldenummer: 01118218.5
(22) Anmeldetag: 28.07.2001
(51) Int. Cl.: C07J 9/00, C11B 13/00

(54) **Verfahren zur Gewinnung von Sterinen aus fettsäurehaltigen Rückständen**
Process for the isolation of sterols from the residues of fatty-acid or methyl-ester production
Procédé d'isolation des stérols de résidus de la production des acides gras ou méthyl-esters

(30) Priorität: 07.08.2000 DE 10038442
(43) Veröffentlichungstag der Anmeldung: 13.02.2002
(73) Patentinhaber: Cognis IP Management GmbH, 40589 Düsseldorf (DE)
(72) Erfinder: Schwarzer, Jörg, Dr., 40723 Hilden (DE); Gutsche, Bernhard, Dr., 40724 Hilden (DE); Wollmann, Gerhard, Dr., 40723 Hilden (DE)

(56) Entgegenhaltungen:
- WO-A-94/05650
- DE-C1- 19 916 034
- US-A- 5 670 669
- US-A- 5 908 946

## Beschreibung

### Gebiet der Erfindung

Die Erfindung befindet sich auf dem Gebiet der Steringewinnung und betrifft ein Verfahren zur Herstellung von Sterinen aus Rückständen der Fettsäureproduktion.

### Stand der Technik

Die Gewinnung von Sterinen aus Destillaten, die bei der Entsäuerung von pflanzlichen Ölen anfallen, oder Destillationsrückständen, die bei der Methylesterproduktion, insbesondere aus der Pflanzenmethylesterherstellung für das Einsatzgebiet "Biodiesel" anfallen, sind in der Patentliteratur bereits verschiedentlich beschrieben worden. Stellvertretend seien hier die Druckschriften EP-A2 0610742 (Hoffmann-LaRoche), GB-A1 2145079 (Nisshin Oil Mills Japan) und EP-A1 0333472 (Palm Oil Research and Development Board) genannt.

Aus dem Europäischen Patent EP-B1 0656894 (Henkel) ist ein Verfahren zur Herstellung von Sterinen bekannt, bei dem man einen Rückstand aus der Methylesterdestillation, der im wesentlichen aus Glyceriden, Sterinen, Sterinestern und Tocopherolen besteht, in Gegenwart alkalischer Katalysatoren mit Methanol umestert. Die Ausbeute an Sterinen läßt jedoch zu wünschen übrig. Sie wird verbessert in einem Verfahren, das die DE 19916034 beschreibt.

Die Ausnutzung und Aufwertung von Rückständen aus der Gewinnung von Fetten und Ölen und deren Bestandteilen schließt jedoch häufig aufwendige Verfahren mit Anwendung umweltschädlicher Lösungsmittel ein. In der Patentschrift US 4148810 werden leichtflüchtige Sterole aus den Destillationsrückständen der Fett- und Ölgewinnung und Aufarbeitung durch Umesterung mit anschließender Lösung in aprotischen, organischen Lösungsmitteln isoliert.

Demzufolge hat die Aufgabe der vorliegenden Erfindung darin bestanden, Sterine in hohen Ausbeuten und in hoher Reinheit durch ein wirtschaftliches Verfahren unter Vermeidung toxikologisch und ökologisch bedenklicher Lösungsmittel herzustellen und dabei Rückstände der Fettsäureproduktion ökonomischer auszunutzen.

### Beschreibung der Erfindung

Gegenstand der Erfindung ist ein Verfahren zur Gewinnung von Sterinen aus den Rückständen der Fettsäure-Produktion, bei dem man
a) in den Rückständen vorhandene freie Fettsäuren durch Polyolyse oder mit einem der niederen Alkohole Methanol oder Ethanol verestert
b) die im Gemisch enthaltenen Partialglyceride bei Temperaturen von 90 bis 145 °C und einem Druck von 2 bis 10 bar über 2 - 20 Minuten mit Methanol oder Ethanol in Gegenwart eines basischen Katalysators umestert,
c) nach der Umesterung den überschüssigen Methanol oder Ethanol aus dem Reaktionsgemisch abdestilliert,
d) den Umesterungskatalysator sowie das gegebenenfalls enthaltene Glycerin abtrennt,
e) den Fettsäurealkylester aus dem Gemisch abdestilliert und
f) die im Sumpf enthaltenen Sterinester und restlichen Partialglyceride durch eine weitere Umesterung bei Temperaturen von 115 bis 145 °C und einem Druck von 2 bis 10 bar über 4 - 8 Stunden in freie Sterine und Fettsäureester überführt.

Es wurde gefunden, dass in den Rückständen der Fettsäureproduktion noch qualitativ hochwertige Sterine enthalten sind, die durch ein Verfahren, in dem eine Veresterung mit zwei Umesterungschritten kombiniert wird, wirtschaftlich und umweltschonend gewonnen werden können. Dabei werden in einem ersten Umesterungsschritt (b) unter milden Bedingungen die Mono-, Di- und Triglyceride, die nach der Veresterung der in den Rückständen enthaltenen freien Fettsäuren auftreten, mit Methanol oder Ethanol in Gegenwart eines basischen Katalysators umgesetzt. Unter den milden Bedingungen bleiben die Sterinester überwiegend noch gebunden, und es bilden sich nur wenig freie Sterine (< 1 Gew.%). Die erste Umesterung verläuft sehr schnell und zeitsparend, sodass sie in einem einfachen Rohrreaktor durchgeführt werden kann.
Nach Abtrennung des überschüssigen Alkohols, Umesterungskatalysators und Glycerins erfolgt eine Destillation der freien Methylester, die die Aufkonzentrierung der Sterinester im Sumpf zur Folge hat. Diese werden jetzt in einen zweiten Umesterungsschritt (f) unter extremeren Bedingungen in freie Sterine umgeestert. Dadurch, dass bei diesem Umesterungsschritt bereits die Begleitstoffe wie Fettsäure, Methylester und Glycerin abgetrennt sind und die Sterinester in aufkonzentrierter Form vorliegen, können die freien Sterine unter wesentlich ökonomischeren Bedingungen gewonnen werden.
Im Anschluß an den zweiten Umesterungsschritt werden die freien Sterine kristallisiert und gewaschen. Sie können dann aufgeschmolzen werden und über einen Fallturm mit Vertropfungseinrichtung und Luft im Gegenstrom in staubfreie , kugelförmige Partikel zur Erstarrung gebracht werden.

Das Verfahren ist für unterschiedliche Ausgangsgemische geeignet. Es werden Ausbeuten in hoher Qualität erreicht. Die zusätzliche Ausnutzung der Destillationsrückstände führt zu einem wirtschaftlich und ökologischen Verfahren, das im technischem Maßstab einfach durchführbar ist.

### Rückstände der Fettsäureproduktion

Untersuchungen im Bereich der Fettsäuregewinnung haben gezeigt, dass die Destillationsrückstände der Fettsäure-Produktion noch über qualitativ gute Sterine, überwiegend in Form von Sterinestern verfügen. Besonders geeignet sind dabei die Rückstände aus der Gewinnung von Soja-, Sonnenblumen- und Rapsfettsäuren, da die Öle eine höhere Ausgangskonzentration an Sterinen haben. Mit zusätzlichem Aufwand können aber auch Destillationsrückstände aus der Gewinnung von Kokos-, Palm- oder Palmkemfettsäuren genutzt werden.
Freie oder auch gebundene Sterine sind nicht wasserlöslich und liegen nach der Spaltung der Fette und Öle, die überwiegend bei 250 - 260 °C und 50 - 60 bar ohne Katalysator erfolgt, zusammen mit der Spalt-Fettsäure und den nicht gespaltenen Partialglyceriden vor. Bei der Gewinnung der freien Fettsäuren erfolgt dann üblicherweise eine Überkopfdestillation der Fettsäuren gegebenenfalls gekoppelt mit einer Fraktionierung (G. Dieckelmann, I.J. Heinz, The Basics of Industrial Oleochemistry (1988, S. 52 ff.). Dabei findet eine zumindest partielle autokatalytische Veresterung der freien Sterine mit freien Fettsäuren zu Sterinestern ebenso wie die Umsetzung von Partialglyceriden und Spuren freien Glycerins statt. Die hohe thermische Belastung bei der Fettspaltung und Aufarbeitung tragen nicht zur Minderung der Produktqualität der Sterine bei.
Trotzdem werden normalerweise diese anfallenden Rückstände aus der Gewinnung von Pflanzenfettsäuren als Tierfutter verwendet. Die Gewinnung der darin anfallenden Sterine stellt daher eine bessere und wirtschaftlichere Ausnutzung der Rohstoffquellen dar.

Typische Konzentrationen an freien und gebundenen Sterinen in den Fettsäuredestillationsrückständen sind:
- aus Sojaöl:: 5-20 Gew%
- aus Sonnenblumenöl:: 5-25 Gew %
- aus Rapsöl:: 5-30 Gew %
- aus Kokos/Palmkernöl:: 5-15 Gew. %
- aus Palmöl:: 3-10 Gew. %

### Verfahrensschritte zur Aufarbeitung der Destillationsrückstände vor dem ersten Umesterungsschritt

### Einsatz von Rückständen mit Restfettsäuren zur Steringewinnung, Veresterung (a)

In einem erfindungsgemäßen ersten Verfahrensschritt werden fettsäurehaltige Rückstände nach der Destillation von gespaltenem Fetten und Ölen mit einer Säurezahl von SZ = 20 - 100, vorzugsweise SZ = 30 - 50 eingesetzt.
Dies sind insbesondere Rückstände der Spaltung von Sojaöl, Sonnenblumenöl, Rapsöl und Kokosöl.
Es können jedoch auch Rückstände der Palmöl-, Palmkernöl-, Baumwollsaatöl-, Maiskeimöl- oder Kokosölverarbeitung eingesetzt werden.
Die Rest-Fettsäuren in den Rückständen werden erfindungsgemäß zuerst verestert oder abgetrennt. Als Veresterungsverfahren kommen in Betracht.
- Polyolyse mit einem mehrwertigen kurzkettigen Alkohol
- Veresterung mit einem der einwertigen Alkohole Methanol oder Ethanol, dabei wird insbesondere der einwertige Alkohol eingesetzt, der auch anschließend in der Umesterung verwendet wird.

Vorzugsweise ist das Veresterungsverfahren *erfindungsgemäß* eine Polyolse. Als Polyol wird vorzugsweise Glycerin eingesetzt. Die Polyolyse mit Glycerin läuft mit und ohne Katalysator bei erhöhten Temperaturen von 180 - 230 °C. Für die Polyolse sind alle Veresterungskatalysatoren denkbar. Bei Fahrweise mit Katalysator wird vorzugsweise ein zweiwertiger Zinn-Katalysator, beispielsweise Zinn-Oxid, Zinn-Oxalat, Zinn-Octoat und Zinn -Chlorid, mit 0,1 - 1 Gew. %, insbesondere 0,2 - 0,4 Gew. % eingesetzt.
Vorzugsweise wird jedoch kein Katalysator zugesetzt.

Als Glycerin wird vorzugsweise die gleiche molare Menge eingesetzt, wie Fettsäuren vorliegen. Damit werden bei der Glycerinolyse bevorzugt Monoglyceride gebildet. Dadurch wird auch ohne Katalysator eine hohe Reaktionsgeschwindigkeit, d.h. kurze Reaktionszeit erreicht. Das bei der Reaktion entstehende Wasser wird abdestilliert. Um einen hohen Umsatz zu erzielen, wird bei End-Temperaturen bis 215 - 230 °C gefahren. Der Druck wird von anfänglich 1 bar bis 5 mbar abgesenkt. Es werden Restsäurezahlen von SZ = 0,5 - 2,5 angestrebt.

Durch die Polyolse mit Glycerin werden die Fettsäuren zu höhersiedenden Partialglyceriden umgesetzt. Das hat den Vorteil, dass gegen Ende der Reaktion nicht umsetzbare UV-Anteile unter Vakuum bei 10 bis 1 - 5 mbar selektiv durch Destillation abgetrennt werden können.

Alternativ sind alle Veresterungsverfahren geeignet, durch die sich die Säurezahl bis kleiner SZ = 4 senken läßt.
Dies sind beispielsweise:
- Homogene Veresterung der Restfettsäuren mit Methanol oder Ethanol mit einem Veresterungskatalysator wie z.B. NaHSO₄, Zinkseifen, Phosphorige Säure H₃PO₃, Phosphorsäure H₃PO₄, P₂O5, para-Toluolsulfonsäure, Alkylbenzolsulfonsäure, Ca-Acetat, Ba-Acetat
- Homogene Veresterung der Restfettsäuren mit Polyolen z.B. Trimethylolpropan, Pentaerythrit ohne Katalysator und/oder Veresterungskatalysatoren wie z.B. zweiwertige Zinnkatalysatoren (s.oben).
- Heterogene Veresterung der Restfettsäuren mit Methanol oder Ethanol, oder Polyolen am katalytisch wirksamen Festbett z.B. mit sauren lonenaustauschern

Gegebenenfalls wird nach der Polyolse bzw. der Veresterung nach einem der oben genannten Verfahren die noch vorhandene Restfettsäure und der Katalysator abgetrennt.
Dabei wird beispielsweise mit einem Natrium-Metasilikat in dem Na₂O gebunden ist, die Restfettsäure neutralisiert und der Katalysator gefällt. Vorzugsweise wird als Natriummetasilikat das unter dem Handelsnamen bekannte Simet -AP (CAS 6834-92-0) in einer Menge proportional der Restsäurezahl (Menge Simet = SZ g/kg Produkt) eingesetzt.
Der Simet-Seifen-Komplex wird durch Filtration oder Zentrifugieren abgetrennt.

### Erster Umesterungsschritt (b)

### Milde Umesterung der Partialglyceride

Im Verfahrensschritt "Milde Umesterung der Partialglyceride" werden Glyceride mit Sterolester mit einer niedrigen Säurezahl eingesetzt. Mit einem der kurzkettigem Alkohole Methanol oder Ethanol werden vorzugsweise nur die Tri- Di- und Monoglyceride unter relativ milden Bedingungen zu Methylester und Glycerin umgesetzt. Unter den milden Bedingungen bleiben die Sterinester im wesentlichen noch gebunden. Es bilden sich nur wenig freie Sterine (kleiner 1 %).

Als Rohstoff für die Umesterung der Partialglyceride werden eingesetzt:
- Reaktionsprodukte der "Polyolse von freien Fettsäuren" nach dem oben genannten Verfahren.
- Reaktionsprodukte der "Veresterung von freien Fettsäuren" nach einem der oben genannten Verfahren.
Dabei werden als Rohstoffe Destillationsrückstände aus der Gewinnung von Soja-, Sonnenblumen-, Raps- ,Kokos- ,Palmkern-, Maiskeim-, Baumwollsaat- oder Palm-Fettsäuren oder deren Mischungen einsetzt.

Als Alkohol für die milde Umesterung wird *vorzugsweise* Methanol mit 15 - 30 Gew % des Rückstandsanteil eingesetzt. Als Katalysator sind alle Umesterungskatalysatoren geeignet. *Vorzugsweise* wird 30 Gew.% ige methanolische Natrium-Methylat-Lösung mit 0,1 - 0,5 Gew. % des Rückstandsanteils eingesetzt.
Die Umesterungsreaktion läuft bei milden Bedingungen ab. Diese können durch die Reaktionstemperaturen oder die Zeitdauer der Reaktion eingestellt werden.
Bevorzugt sind Temperaturen von T = 90- 145 °C, insbesondere 115 - 130°C mit einem Druck von p = 2 - 10 bar, insbesondere 3 - 5 bar, innerhalb von 2 - 20 Minuten. Als Reaktoren werden Batch-Rührautoklaven als auch kontinuierliche Reaktoren wie z.B. turbulent durchströmte Rohrreaktoren eingesetzt (EP 0494177B1).

### Aufarbeitungsschritte zwischen dem ersten und zweiten Umesterungsschritt

### Abdestillieren des Methanols (c)

Im Verfahrensschritt "Abdestillieren des Überschuss Methanols" wird das heiße Reaktionsgemisch aus Schritt b) in eine Vorlage entspannt. Dabei destillieren 55 - 85 % des Überschussmethanols ab. Das System kühlt sich auf 65 -85 °C ab. Das noch im Reaktionsprodukt verbleibende Restmethanol wird vorzugsweise nicht mehr abdestilliert. Es dient als Lösungsvermittler bei der nachfolgenden Stufe.

Vorzugsweise wird vor oder während dem Flashen zur Inaktivierung des alkalischen Katalysators Wasser in das System gegeben. Vorzugsweise wird molar genausoviel Wasser zugegen wie alkalischer Katalysator eingesetzt wurde.

### Katalysatorfällung und Abtrennung der Metalle(d)

In den Rohstoffen z.B. den Rückständen der Fettsäure-Destillation sind noch Metalle wie Calcium 40-100 ppm , Eisen 20 - 1000 ppm, Phosphor bis 15 ppm enthalten.
In den Reaktionsprodukten der "Veresterung von freien Fettsäuren" sind noch Veresterungs-Katalysatoren enthalten; nach der Polyolyse mit Katalysator vorzugsweise zweiwertige Zinn-Verbindungen.
Diese Katalysator-Seifen sind im Reaktionsgemisch der ersten Umesterung löslich. Um sie abtrennen zu können, werden sie erfindungsgemäß mit Säuren in unlösliche Verbindungen umgewandelt und gefällt. Als Säuren werden vorzugsweise wässrige Lösungen von Zitronensäure oder Phosphorsäure eingesetzt. Als Menge der Säure wird vorzugsweise die einfache bis doppelte molare Menge der Metall-Konzentration eingesetzt.
Nach dem Fällen wird der ausgefallene, metallhaltige Schlamm durch Zentrifugieren und Auswaschen mit Wasser abgetrennt. Alternativ werden die gefällten Metalle adsorbiert. Als Adsorbentien eignen sich amorphe mit organischen Säuren beladene Silicagele wie z.B. Trysil-Typen der Fa. Grace.
Es werden Rest- Metallgehalte von 0 - 6 ppm erreicht.

Alternativ erfolgt die Metallfällung und Abtrennung aus Rückständen der Fettsäuredestillation direkt nach der Polyolse ohne Katalysator zusammen mit der Abtrennung des Simet-Seifen-Komplex durch Filtration oder Zentrifugieren.

### Glycerinabtrennung und Wäsche (d)

Anschließend erfolgt eine Abtrennung des Glycerins zum Beispiel durch Dekantieren. Alternativ wird aus dem Reaktionsprodukt das freie Glycerin und das Rest-Methanol mit Wasser (zwei bis dreimal 10 Gew. % Wasser pro Reaktionsprodukt) in Gegenwart von Citronensäure zur Emulsionsspaltung ausgewaschen. Das Produkt wird anschliessend getrocknet

### Methylester-Destillation (e)

Zur Anreicherung der Sterinester wird der Methylester abdestilliert. Dabei wird bei Temperaturen von 170 - 200 °C und Drücken von 1 - 5 mbar destilliert.
*Erfindungsgemäß* sind in dem Verfahrensschritt "Milden Umesterung der Partialglyceride" im wesentlichen nur die Partialglyceride umgeestert worden. Da die Sterine noch größtenteils als Sterinester vorliegen, sind sie höhersiedend und werden bei der Destillation des Methylesters nicht abdestillliert. Sie verbleiben *erfindungsgemäß* vollständig als angereichertes Wertprodukt im Sumpf und werden nicht als Verluste mit dem Methylester abdestilliert.

### Zweiter Umesterungsschritt (f)

### Umesterung der Sterinester

Im Sumpfprodukt der Fettsäureester-Destillation sind die Sterinester angereichert. Sie werden durch Umesterung mit einem kurzkettigen Alkohol, vorzugsweise Methanol, in Gegenwart eines Katalysators in freie Sterine umgesetzt. Da die Umesterung von Sterinestern unter verschärfteren Bedingungen ablaufen muss als die Umesterung von Partialglyceriden, sind höhere Alkohol- und Katalysatormengen sowie längere Reaktionszeiten nötig.

Die zugesetzte Alkoholmenge beträgt 20 - 100 Gew.% des Sumpfproduktes der Fettsäureester-Destillation. Bei Methanol als Umesterungsreagens wird vorzugsweise 40 - 60 Gew.%, vorzugsweise 45 - 55 Gew. % des Sumpfproduktes der Fettsäureester-Destillation eingesetzt. Als Katalysator sind auch hier alle Umesterungskatalysatoren geeignet.

Die Reaktion läuft bei Temperaturen von 115 - 145°C, insbesondere 120 - 130°C und einem Druck von 2 - 10 bar, insbesondere 3 - 6 bar innerhalb von 3 - 10 h, insbesondere 6 - 8 h ab. Als Katalysator sind alle Niederdruck-Umesterungskatalysatoren geeignet. Vorzugsweise wird 30%ige methanolische Natrium-Methylat-Lösung mit 0,3 - 5 Gew.%, insbesondere 1 - 3 Gew. % des Rückstandes der Fettsäureester-Destillation eingesetzt. Als Reaktor können beispielsweise Batchrührautoklaven eingesetzt werden.

### Nachgeschaltete Verfahren zur Anreicherung von Sterinen

Aus den Anmeldungen DE 4228476 und DE 19916034. sind nachgeschaltete Verfahren zur Anreicherung von Sterinen bekannt. Dazu zählen weitere Schritte wie
- Katalysatorabtrennung und Wäsche mit Wasser
- Kristallisation der Sterine, Abtrennen der Mutterlauge
- Wäsche der Kristalle
- Konfektionieren der Sterine, durch Aufschmelzen der Kristalle und Konfektionieren
Im Verfahrensschritt "Konfektionieren der Sterine" werden die Sterine aufgeschmolzen und bei 140-155 °C entweder über eine Schuppenwalze verschuppt oder erfindungsgemäß über eine Vertropfungseinrichtung in einen Fallturm vertropft und im Gegenstrom mit gekühlter und/oder getrockneter Luft soweit gekühlt, dass sie als staubfreie Perlen erstarren.

### Beispiele

### Beispiel 1:

In einem Rührbehälter wurden 2600 g Destillationsrückstand der Spaltung von Sonnenblumenöl mit SZ = 37 mit 244 g Glycerin und keinem Katalysator umgesetzt. Nach Erreichen von 220 °C und einem Vakuum von 50 mbar wies die Probe SZ = 5,9 auf. Durch weiteres Absenken des Vakuums auf 5 mbar und Erhöhen der Temperatur auf 225 °C sank die Säurezahl auf SZ = 0,2 ab.

### Beispiel 2:

In einem Rührbehälter wurden 2000 g Destillationsrückstand der Spaltung von Sonnenblumenöl mit SZ = 53 mit 174 g Glycerin und 8,6 g Zinn(2) isooctoat umgesetzt. Nach Erreichen von 215 °C und einem Vakuum von 7 mbar wies die Probe SZ = 2,7 auf.

### Beispiel 3:

In einem Rührbehälter nach Beispiel 1 wurden 2700 g Destillationsrückstand der Spaltung von Sojaöl mit SZ = 25 mit 157 Glycerin und keinem Katalysator umgesetzt. Nach Erreichen von 220 °C und einem Vakuum von 50 mbar wies die Probe SZ = 1,7 auf.
Nach Zugabe von 4,9 g Simet AP, 4,9 g Trysil 300 und 4,9 g Wasser und abfiltrieren des Schlammes wies das Reaktionsprodukt SZ = 1,1 auf.

### Beispiel 4.

In einem Rührbehälter nach Beispiel 1 wurden 2110 g Destillationsrückstand der Spaltung von Sojaöl mit SZ = 33 mit 114 g Glycerin und 8,9 g Zinn(2) isooctoat umgesetzt. Nach Erreichen von 215 °C und einem Vakuum von 7 mbar wies die Probe SZ = 2,2 auf. Mit dem Wasser wurden UV-Anteile mitabdestilliert.

### Beispiel 5:

In einem Rührbehälter wurden 1000 g Destillationsrückstand der Spaltung von Sojaöl mit SZ = 33 mit 200 g Methanol und 10 g 50 % ige H₃PO₃ unter Kochen im Rückfluss umgesetzt. Bei leichtem
Überdruck von 3 bar und T = 135 °C sank die Säurezahl bis SZ = 4 ab.

### Beispiel 6:

2070 g Reaktionsprodukt aus Beispiel 4 wurde in einem Autoklav mit 310 g Methanol und 31 g 30 % iger Na-Methylat-Lösung bei 137 °C und 6 bar 8 Minuten lang mild umgeestert. Es bildeten sich bei der milden Umesterung nur 0,8 % freie Sterine. Die restlichen Sterine blieben verestert.
Nach Entspannen des Reaktionsgemisches flashte 135 g Methanol ab.

Das Reaktionsgemisch wurde mit 30 g 50 % iger Zitronensäure neutralisiert und dreimal mit 10 % Wasser neutral gewaschen.
Nach der Trocknung wurde 983 g Methylester bei 180 °C und 1 mbar abdestilliert. Das Methylester-Destillat war frei von Sterin. Es traten keine Sterin-Verluste bei der Destillation auf. Die Sterinester blieben im Rückstand II.
130 g Rückstand II der Methylesterdestillation wurde mit 65 g Methanol und 3,2 g 30 % ige Na-Methylatlösung 8 h bei 120 °C und unter Niederdruck von 5 bar umgeestert und danach 25 g Methanol abgeflasht.
Nach Neutralisation des alkalischen Katalysators mit Citronensäure und Wäsche des Reaktionsproduktes mit Wasser, wurden 130 g Produkt mit 7,5 % freien Sterinen und 0,04 % gebunden Sterinen in die Kristallisation bei 15 °C eingesetzt. Durch Filtration wurden 104 g Mutterlauge abgetrennt.
Der Filterkuchen wurde mit 65 g Methylester, 65 g Methanol und 30 g Wasser gewaschen. Der getrocknete Filterkuchen enthielt 93,9 GC-Flächen % freie Sterine, kleiner 0,3 % Methylester C18 und keine gebundenen Sterine.
Er wies auf: 1,2 % Cholesterin, 1,8 % Brassicasterin, 23,1 % Campesterin, 15,3 % Stigmasterin, 48,9 % β-Sitosterin, 2,2 % 5 Avenasterin, 1 % Stigmasterin, 0,3 % 7 Avenasterin und 0,05 % Citrostadienol.

### Beispiel 7:

Raps - Sterine wurden aufgeschmolzen und unter Stickstoffdruck von 0,3 bar bei 150 °C über eine 650 µm Düse in einen 24 m hohen Fallturm mit 1 kg/h/Düse vertropft und im Gegenstrom mit 15 °C kalter Luft gekühlt und erstarrt. Es bildeten sich staubfreie Perlen. Die Endprodukttemperatur betrug 22 °C.

### Beispiel 8:

Raps - Sterine wurden aufgeschmolzen und unter Stickstoffdruck von 0,35 bar bei 155 °C über eine 800 µm Düse in einen 24 m hohen Fallturm mit 3 kg/h/Düse vertropft und im Gegenstrom mit 13 °C kalter Luft gekühlt und erstarrt. Es bildeten sich staubfreie Perlen. Die Endprodukttemperatur betrug 35 °C.

## Patentansprüche

1. Verfahren zur Gewinnung von Sterinen aus Rückständen der Fettsäure- und/oder der Methylesterproduktion, **dadurch gekennzeichnet, dass** man
a) in den Rückständen vorhandene freie Fettsäuren mit einem mehrwertigen Alkohol, vorzugsweise Glycerin, oder einem der niederen einwertigen Alkohole Methanol oder Ethanol verestert, danach
b) die im Gemisch enthaltenen Partialglyceride bei Temperaturen von 90 bis 145 °C und einem Druck von 2 bis 10 bar über 2 - 20 Minuten mit Methanol oder Ethanol in Gegenwart eines basischen Katalysators umestert,
c) nach der Umesterung das überschüssige Methanol oder Ethanol aus dem Reaktionsgemisch abdestilliert,
d) den Umesterungskatalysator sowie das gegebenenfalls enthaltene Glycerin abtrennt,
e) den Fettsäurealkylester aus dem Gemisch abdestilliert und
f) die im Sumpf enthaltenen Sterinester und restlichen Partialglyceride durch eine weitere Umesterung bei Temperaturen von 115 bis 145 °C und einem Druck von 2 bis 10 bar über 4 - 8 Stunden in freie Sterine und Fettsäureester überführt.

2. Verfahren nach Anspruch 1, **dadurch gekennzeichnet, dass** man die gewonnenen freien Sterine durch nachfolgende Kristallisation und Wäschen aufreinigt.

3. Verfahren nach den Ansprüchen 1 und 2, **dadurch gekennzeichnet, dass** die Sterine durch Verschuppen oder Vertropfen konfektioniert werden.

4. Verfahren nach den Ansprüchen 1 bis 3, **dadurch gekennzeichnet, dass** man zur weiteren Aufarbeitung die Sterinkristalle aufschmilzt und über einen Fallturm mit Vertropfungseinrichtung und Luft im Gegenstrom in staubfreie, kugelförmige Partikel erstarrt.

5. Verfahren nach den Ansprüchen 1 bis 4, **dadurch gekennzeichnet, dass** man Destillationsrückstände der Fettsäureproduktion pflanzlicher Herkunft einsetzt.

6. Verfahren nach den Ansprüchen 1 bis 5, **dadurch gekennzeichnet, dass** man Destillationsrückständen aus der Gewinnung von Soja-, Sonnenblumen-, Raps-, Kokos-, Palmkern- oder Palmfettsäuren oder deren Mischungen einsetzt.

## Claims

1. process for the production of sterols from the residues of fatty acid production and/or methyl ester production, in which
a) free fatty acids present in the residues are esterified with a polyhydric alcohol preferably glycerol or one of the lower monohydric alcohols methanol or ethanol, thereafter
b) the partial glycerides present in the mixture are transesterified with methanol or ethanol in the presence of a basic catalyst for 2 to 20 minutes at temperatures of 90 to 145°C and under a pressure of 2 to 10 bar,
c) the excess methanol or ethanol is distilled off from the reaction mixture after the transesterification,
d) the transesterification catalyst and the glycerol present, if any, are removed,
e) the fatty acid alkyl ester is distilled off from the mixture and
f) the sterol esters and residual partial glycerides present in the bottom product are converted into free sterols and fatty acid esters by further transesterification for 4 to 8 hours at temperatures of 115 to 145°C and under a pressure of 2 to 10 bar.

2. A process as claimed in claim 1, **characterized in that** the free sterols obtained are purified by subsequent crystallization and washing.

3. A process as claimed in claims 1 and 2, **characterized in that** the sterols are converted into flakes or drops.

4. A process as claimed in claims 1 to 3, **characterized in that**, for further working up, the sterol crystals are melted, sprayed through droplet-forming nozzles into a gravity tower and solidified by cooling with air flowing in countercurrent to form dust-free spherical particles.

5. A process as claimed in claims 1 to 4, **characterized in that** fatty acid distillation residues of vegetable origin are used.

6. A process as claimed in claims 1 to 5, **characterized in that** distillation residues from the production of soybean, sunflower, rapeseed, coconut, palm kernel or palm oil fatty acids or mixtures thereof are used.

## Revendications

1. Procédé d'obtention de stérols à partir de résidus de la production d'acides gras et/ou d'esters méthyliques,
**caractérisé en ce qu'**
a) on estérifie les acides gras libres présents dans les résidus avec un alcool polyvalent, de préférence du glycérol, ou l'un des alcools inférieurs monovalents, méthanol ou éthanol, puis
b) on transestérifie les glycérides partiels contenus dans le mélange à des températures de 90 à 145°C et sous une pression de 2 à 10 bars pendant 2 à 20 minutes avec du méthanol ou de l'éthanol en présence d'un catalyseur basique,
c) après la transestérification on élimine le méthanol ou l'éthanol excédentaire du mélange réactionnel par distillation,
d) on sépare le catalyseur de transestérification ainsi que le glycérol, éventuellement présent,
e) on élimine l'ester alkylique d'acide gras du mélange par distillation et
f) on transforme les esters stériques contenus dans le bas de colonne et les glycérides partiels restants en stérols libres et en esters d'acides gras par une étape de transestérification ultérieure à des températures de 115°C à 145°C et sous une pression de 2 à 10 bars pendant 4 à 8 heures.

2. Procédé selon la revendication 1,
**caractérisé en ce qu'**
on purifie les stérols libres obtenus par des étapes de cristallisation et de lavage ultérieures.

3. Procédé selon les revendications 1 et 2,
**caractérisé en ce qu'**
on met les stérols en forme par écaillage ou gouttage.

4. Procédé selon les revendications 1 à 3,
**caractérisé en ce que**
pour les purifier de manière plus poussée, on fait fondre les cristaux de stérol et on les solidifie sous forme de particules sphériques exemptes de poussière en utilisant une colonne de chute avec un dispositif de gouttage et de l'air à contre-courant.

5. Procédé selon les revendications 1 à 4,
**caractérisé en ce qu'**
on utilise des résidus de distillation de la production d'acides gras d'origine végétale.

6. Procédé selon les revendications 1 à 5,
**caractérisé en ce qu'**
on utilise des résidus de distillation provenant de la préparation d'acides gras de soja, de tournesol, de colza, de coco, de palmiste ou de palme ou leurs mélanges.
